# EUROPEAN PATENT SPECIFICATION

(11) **EP 0 290 138 B1**
(45) Date of publication and mention of the grant of the patent: **17.07.1996**
(21) Application number: 88303073.6
(22) Date of filing: 06.04.1988
(51) Int. Cl.: A61B 17/56

(54) **Method of manufacturing of anatomical precontoured plates**
Verfahren zur Herstellung von anatomisch vorgeformten Knochenplatten
Méthode de fabrication des plaques à conformation anatomique

(30) Priority: 07.04.1987 US 35658
(43) Date of publication of application: 09.11.1988
(73) Proprietor: POLLOCK, Richard Allison, Duluth, Georgia 30136-6427 (US)
(72) Inventor: POLLOCK, Richard Allison, Duluth, Georgia 30136-6427 (US)
(74) Representative: Singleton, Jeffrey

(56) References cited:
- EP-A- 0 003 763
- DE-A- 2 806 207
- DE-A- 3 434 807
- FR-A- 2 472 373

## Description

This invention relates to a method of manufacturing osteosynthesis plates for alignment and stabilization of fractured bones.

### BACKGROUND OF THE INVENTION

Repair of dislocated bone involves two primary steps: realignment of the dislocated fragments or segments and stabilization of the bone. Dressings such as plaster or oil-soaked linen and wire have been used since antiquity for bone stabilization, but wire emerged as the prevalent appliance for bone stabilization in the nineteenth century.

Placement of wire loops through holes drilled in bone has long been an accepted technique, but more recent experience, during and after World War II, suggested that bone repaired with wire loops is not rigidly stable. The advent of mechanization and concomitant faster travel resulted in high velocity injuries more severe in nature than those previously encountered. Experience in treatment of such injuries has made it evident that bone fragments connected by wire loops are free to hinge along the fracture line. This bone fragment mobility interferes with healing and results in delayed recovery, skeletal deformity including midfacial shortening, and high rates of infection. Patients with complex injuries have often been crippled or disfigured for life, and many such injuries have been fatal.

Crude metal plates were introduced in Europe in approximately 1957 for the repair of orthopedic fractures. These plates, which were secured to bone with screws, advantageously prevented the bone fragment mobility that is often associated with wire-stabilized injuries. Subsequent generations of plates and fasteners, together with new instruments, allowed rigid stabilization of orthopedic and craniofacial fractures to become a reality. Complex, comminuted and severely dislocated fractures could then be effectively treated.

Typical of plates presently provided for cranial and facial osteosynthesis include those of the Wurzburg, Steinhauser and Champy systems sold in the United States by Walter Lorenz Surgical Instruments, Inc., Jacksonville, Florida, plates of Synthes, Inc., Basel, Switzerland and Paoli, Pennsylvania, and compression plates provided by Howmedica International, Inc., Kiel, Federal Republic of Germany. These plates present common, severe disadvantages, however.

Such plates typically comprise small, generally flat, elongated sections of metal. The sections contain round and perhaps elongated screw holes at various points along their lengths for fastening the sections to bone. The sections may be linear, curved, T-shaped, L-shaped or otherwise angled in their generally planar dimensions for positioning on various portions of the skeleton.

Because no surface of the human skeleton is flat, existing plates must be extensively twisted, formed and bent during surgery to conform to portions of the skeleton on which they are to be affixed. During a six to eight hour surgical procedure, as much as 30 to 45 minutes of time may be expended shaping and re-shaping metal plates. This additional time increases anesthesia requirements and operating room time and increases the potential of infection.

The inevitable over-bending and under-bending of plates during efforts to form the plates during surgery creates crimps and other surface imperfections in the plates and it alters their structural integrity due to metal fatigue. Surface imperfections can also irritate overlying tissue. Weakened structure due to excessive bending and twisting in the operating room is of paramount importance because it can lead to structural failure later; these plates frequently must remain in patients' faces for the rest of their lives and must undergo tremendous stresses, as for instance in the mandibular or ramus areas.

DE-B-2603087 describes a method of manufacturing a plate for setting symphysis fractures which is contoured in three dimensions and which may be made by forming a sheet of thermoplastics material.

The heads of screws provided with present plates extend beyond the plane of the outer surfaces of the plates end create voids between bone and the periosteal lining. The contour of screw heads and often the plates are frequently transmitted through overlying soft tissue and thereby made visible on the patient's face; both screw heads and plates therefore frequently may be palpated beneath the skin's surface.

The planar nature of conventional plates increases this problem of transmission through the facial soft tissue. Rather than having edges which conform to and grip the bone, the edges of present plates frequently form a tangent with respect to the skeleton so that the patient may actually be able to push subcutaneous tissue between the plate edge and bone with his or her fingernail.

The invention provides a method of manufacturing an osteosynthesis plate as set out in the appended claim.

The invention takes advantage of the surprising fact that human adult craniofacial osseous structure and shape are highly similar among the population. Accordingly, plates can be preformed during manufacture to fit a large proportion of the human adult population. Consequently, less time is required during surgery to twist and bend the plates, and their structural characteristics need not be adversely affected by extensive bending, twisting and shaping. Furthermore, they can be made of harder and stiffer material and thus perform more effectively, since they do not need to be soft enough to allow the surgeon to bend and twist them easily in the operating room.

The plates may be packaged and presented for use on forms which simulate portions of the skull, so that their intended cranial or facial position is more easily recognized by members of the surgical team. Because bone thickness at particular craniofacial skeletal locations is highly consistent in the adult population, appropriate length screws may be packaged with the plates with which they are to be used, as for instance, by insertion through or adjacent to the plates on the skull-like packaging platform. Important surgical time is thus saved in selecting screws.

Bone stabilization plates made by a method in accordance with the invention are shaped in three dimensions during manufacturing to require less bending and contouring during surgery in order to save time, minimize surface irregularities in the installed plates and reduce metal fatigue due to bending and twisting.

Bone stabilization plates made by a method in accordance with the invention may be arced in cross-section with respect to their longitudinal axes in order better to grip bone and minimize space between plate and bone.

Bone stabilization plates made by a method in accordance with the invention may transmit fewer and smaller surface irregularities through the soft tissues of the patient's face.

Other objects, features and advantages of the present invention will become apparent with reference to the remainder of the disclosure, claims and drawings.

### BRIEF DESCRIPTION OF THE DRAWINGS

FIG. 1 is a perspective view of craniofacial structure showing installed bone stabilization plates made according to the present invention.

FIG. 2 is a perspective view of craniofacial structure showing additional installed bone stabilization plates made according to the present invention.

FIG. 3 is a side elevational view of craniofacial structure showing other installed bone stabilization plates made according to the present invention.

FIG. 4 is a plan view of a portion of the mandibular angle plate shown in FIG. 3.

FIG. 5 is a cross-sectional view of the plate of FIG. 4 taken along section 5--5 of that plate.

FIG. 6 is a cross-sectional view of the plate of FIG. 4 taken along sections 6-6 of that plate.

FIG. 7 is a plan view of the zygomatic arch plate shown in FIG 3.

FIG. 8 is a cross-sectional view of the plate of FIG. 7 taken along section 8--8 of that plate.

FIG. 9 is a cross-sectional view of the plate of FIG. 7 taken along section 9--9 of that plate.

FIG. 10 is a perspective view of a plate made according to the present invention showing absolute and relative reference systems used in connection with the plates.

FIG. 11A is a front elevational view of a glabellar plate made according to the present invention.

FIG. 11B is a plan view of the plate of FIG. 11A.

FIG. 12A is a front elevational view of a medial canthal plate made according to the present invention.

FIG. 12B is a right side elevational view of the plate of FIG. 12A.

FIG. 13 is a front elevational view of a nasofrontal plate made according to the present invention.

FIG. 14A is a front elevational view of a lateral buttress plate made according to the present invention.

FIG. 14B is a plan view of the plate of FIG. 14A.

FIG. 15 is a face plane view of a plate made by a method according to the present invention having a rectangular configuration for plate segments.

FIG. 16 is a contour plane elevational view of the plate of FIG. 15.

FIG. 17 is a face plane elevational view of a plate made by the present invention having a second, hexagonal shaped segment configuration.

FIG. 18 is a contour plane elevational view of the plate of FIG. 17.

FIG. 19 is a face plane elevational view of a plate made by the present invention with a third, generally circular shaped segment configuration.

FIG. 20 is a contour plane elevational view of the plate of FIG. 19.

### DETAILED DESCRIPTION OF THE DRAWINGS

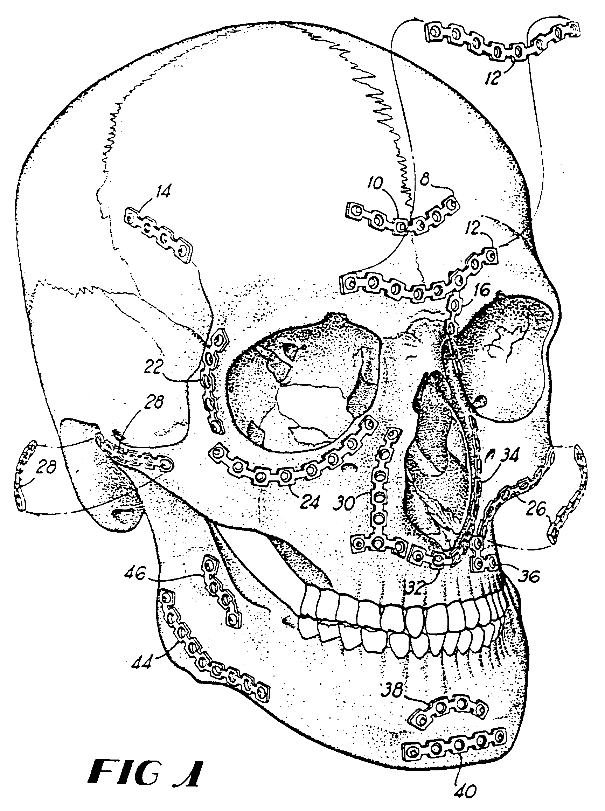

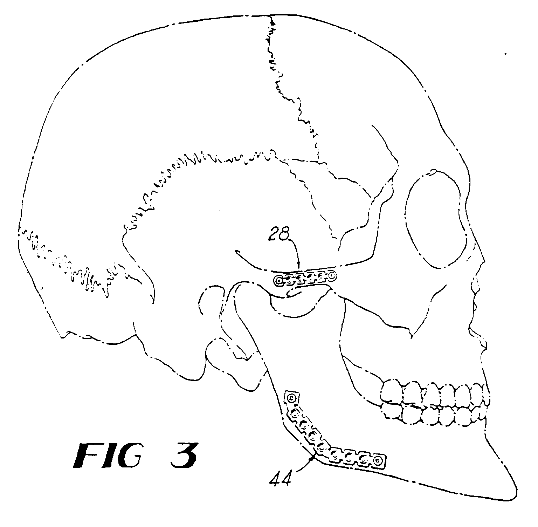

FIGS. 1-3 illustrate configuration and placement of various osteosynthesis or bone stabilization plates 8 according to the present invention. The plates shown in these figures can be generally categorized as falling in the frontal or frontonasal, zygomatic, maxillary and mandibular groups.

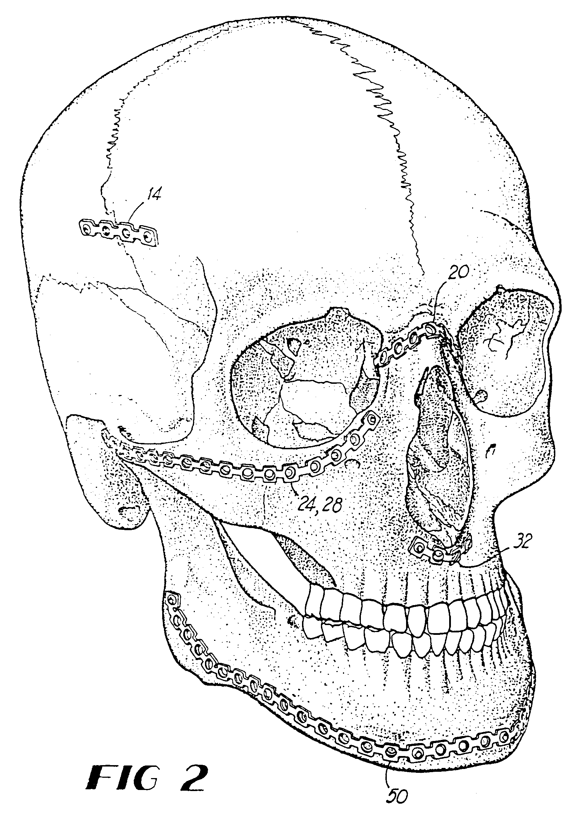

The frontal group contains upper forehead plate 10, glabellar plate 12, panskull plate 14, nasofrontal suspension plate 16, utility plate 18 (not shown) and medial canthal reconstruction plate 20 shown in FIG. 2. The zygomatic group contains frontozygomatic suture plate 22, inferior orbital rim plate 24, lateral buttress plate 26 and zygomatic arch plate 28. The maxillary group contains medial buttress "T" 30, lateral buttress 26, inferior orbital rim (abbreviated) plate 24 (not shown); split palate plate 32, medial buttress "J" plate 34 and "L" plate 36. The mandibular group contains upper symphysis plate 38, marginal symphysis plate 40, interior body plate 42 (not shown), angle plate 44, ridge plate 46, posterior body plate 48 (not shown) and hemimandible plate 50 shown in FIG. 2. These plates are manufactured according to the present invention to be precontoured and secured to locations on the craniofacial skeleton corresponding with their names as generally shown in FIGS. 1-3.

Plates 8 according to the present invention, unlike earlier plates, are manufactured to correspond in three dimensions to their locations on the craniofacial skeleton. An example is lateral buttress plate 26 shown in FIGS. 1 and 13. Unlike earlier osteosynthesis plates, this plate is shaped during manufacture in the form of an "S" in its flat dimension. This dimension is referred to as the "face dimension 52." The plate is arched in the cross-sectional dimension perpendicular to the face dimension to form a convex top face 51 and a concave bottom face 53. This second dimension is referred to as the "cross-sectional dimension 54." Also unlike previous plates, the plate is shaped during manufacture in the third dimension to contour to the portion of the bone to which it will be attached. This third dimension, which is perpendicular to the face dimension 52 and the cross-sectional dimension 54, is referred to as the "contour dimension 56."

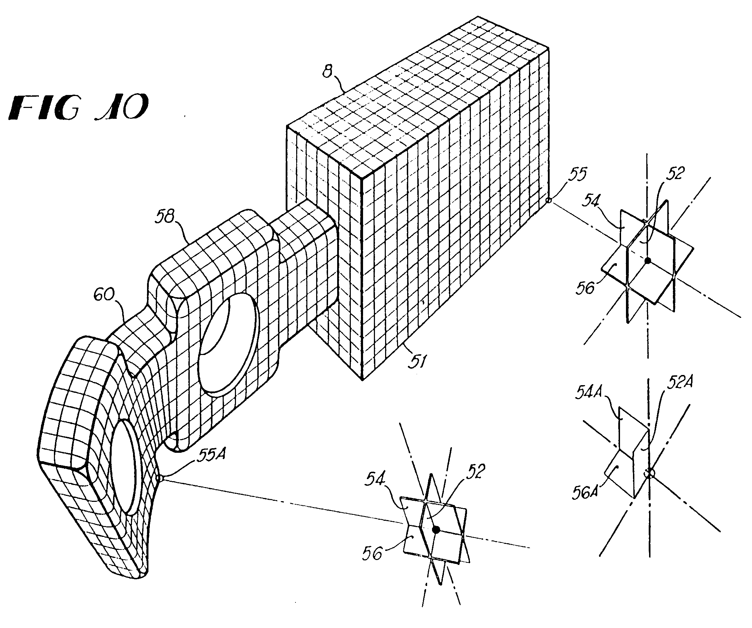

Face dimension 52, cross-sectional dimension 54 and contour dimension 56 are illustrated in FIG. 10 which shows diagramatically a portion of a plate 8 according to the present invention formed from a hypothetical block of material. These dimensions correspond to the orientation of particular points on or within plate 8. Thus, face dimensions 52 with respect to reference point 55 or 55A on top surface 51 of plate 8 is the plane which is tangent to top surface 51 at that point. Cross-sectional dimension 54 is the plane which contains point 55 or 55A, which is orthogonal to face dimension 52 and which contains the cross section of plate 8 at that point. Contour dimension 56 is the plane which is orthogonal to face dimension 52 and cross-sectional dimension 54 at reference point 55 or 55A.

If plate 8 were flat and straight, as are many conventional plates, then face dimension 52, cross-sectional dimension 54 and contour dimension 56 would correspond to face plane 52A, cross-sectional plane 54A and contour plane 56A as shown in FIG. 10. In such a case, face plane 52A would be tangent to all points on top surface 51, and all cross-sectional dimensions 54 of plate 8 would be orthogonal to top surface 51 and face plane 52A. Face dimension 52 and face plane 52A; cross-sectional dimension 54 and cross-sectional plane 54A and contour dimension 56 and contour plane 56A would therefore be coincident. Planes 52A, 54A and 56A thus define an absolute reference system while dimensions 52, 54, and 56 define a reference system that is relative with respect to locations on plates 8.

A conventional plate which must be bend and twisted significantly in the operating room requires deformation of the alignment of the plate's crystal lattices according to the angular differences in three dimensions between relative dimensions 52, 54 and 56 at any point in the plate and absolute planes 52A, 54A and 56A. Such deformation can cause weakness due simply to mechanical alterations in the crystalline structure and due to other effects such as frictional heat generated during bending and twisting. These can adversely affect hardness, stiffness and tensile strength. The present invention can thus minimize weaknesses in the structure of plates 8 which would otherwise be caused by excessive bending, twisting and contouring in the operating room.

Plates 8 can be made of stainless steel, titanium, vitallium (an alloy of cobalt, chromium and molybdenum) or other suitable, even non-metallic, materials. Stainless steel is subject to corrosion when exposed to electrolytes containing hydrogen and oxygen, so installed stainless steel plates may corrode over time and cause localized metallosis. Stainless steel is also subject to contact and friction, or fretting, corrosion. Stainless steel plates with protective coated highly polished surfaces can have better corrosion characteristics, but the surgical team must exercise great care not to damage this surface and affect corrosion potential.

Titanium is a softer metal then stainless steel and is frequently found not to be sufficiently rigid to withstand large forces placed on bone stabilization plates, particularly in the mandibular area. Vitallium is desireable because it can remain in the body for long periods of time without metallosis and need for removal, because it resists fretting, corrosion and oxidation, and because it is a particularly hard alloy.

Plates 8, because they are precontoured and require minimal bending and twisting in the operating room, are particularly well-suited to be made of polymeric, composite or other non-metallic materials which are not as easily shaped at room temperature as are metals. Plates 8 of such materials may also be manufactured to be resorbable in the body; such plates gradually wear away so that any irregularities transmitted through the patient's facial soft tissues diminish over time. Such resorbable materials include, for instance, compounds of polyglycolic acid.

Plates 8 are stamped They may be shaped in the contour dimension 56 during initial stamping or forging, or they may be shaped in a subsequent manufacturing step. After a plate 8 has been shaped or while it is being shaped as desired in all three dimensions, it may be subjected to a hardening or tempering process such as cycles of heating and quenching.

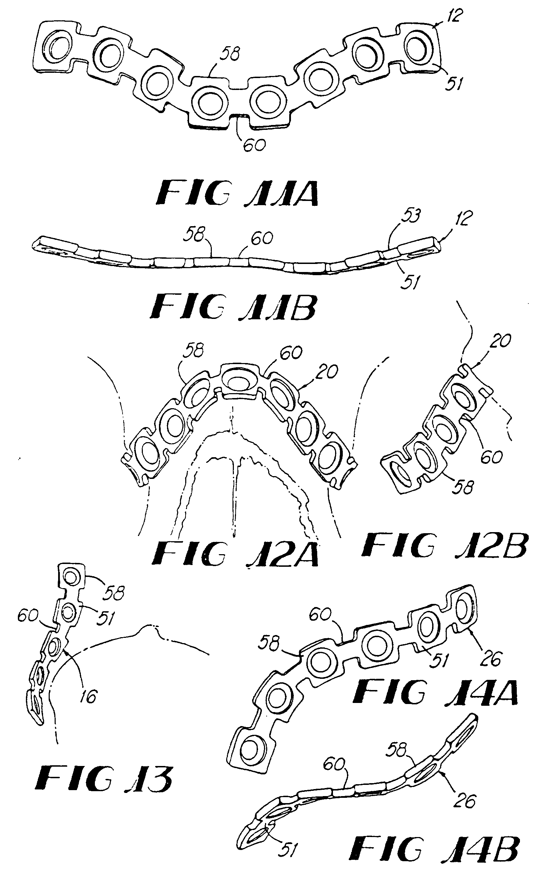

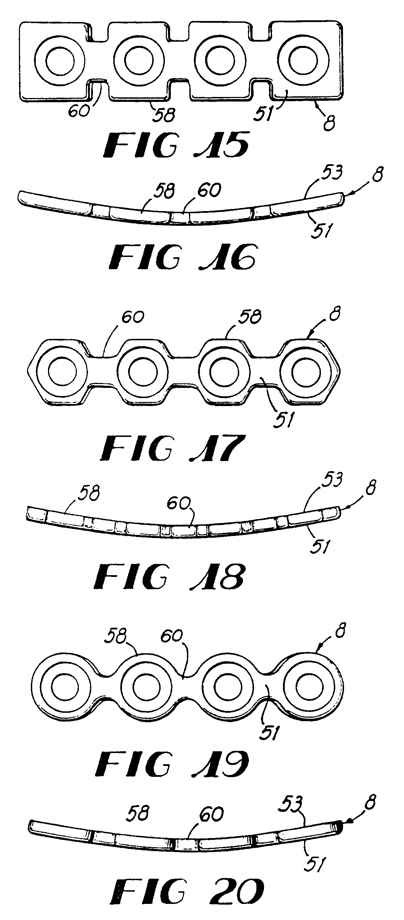

Plates 8 can, but need not, comprise a series of segments 58 connected by throat sections 60. Segments 58 may be generally rectangular as shown in FIGS. 15 and 16, generally hexagonal as shown in FIGS. 17 and 18, generally circular as shown in FIGS. 19 and 20 or of any other desired shape. Alternatively, plates 8 can exclude throat sections 60 so that their sides in the face dimension are continuous, as, for instance, linear, curvilinear or curved.

A plate 8 made by a method according to the present invention is designed so that its cross-sectional moment of inertia or polar moment of inertia is constant. Such a plate has curved sides in its face dimension 52. Those curves, together with the screw hole definitions and the shape of the plate in the cross-sectional dimension, can be configured to provide a uniform polar moment of inertia in the cross-sectional dimension for uniform torsional resistance about the longitudinal axis, or a uniform moment of inertia in the cross-sectional dimension for uniform resistance to bending in the contour dimension. Plates 8 may also be constructed which satisfy both of these conditions for uniform twisting and bending in the contour dimension, and they nay also have uniform moments of inertia in the contour or face dimensions for uniform resistance to bending or twisting or both. Such designs allow the plates to bend more predictably and thus be finally shaped in surgery more easily and quickly. Such moments of inertia may be calculated graphically, using incremental techniques or by any other appropriate method.

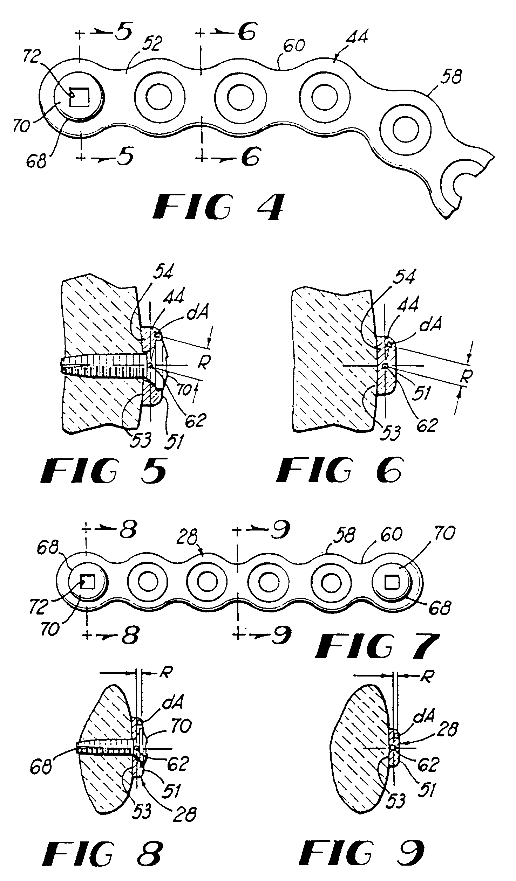

FIGS. 4, 5 and 6 illustrate a plate 8 made by a method according to the present invention with a uniform polar moment of inertia in the cross-sectional dimension 54. FIG. 6 schematically shows the concept of determining the polar moment of inertia, which is the integral of the product of each incremental area dA of the cross section multiplied by the square of its distance R from the center of gravity of the cross section. Because more area is located farther from the center of gravity in a cross section containing a screw hole S as shown, for example, in FIG. 5, the throat 60 cross sectional area as shown in FIG. 6 is larger than the segment 58 cross sectional area.

FIGS. 7, 8 and 9 demonstrate a plate design for uniform resistance to bending in the contour dimension. The moment of inertia about bending axis 66 as shown in FIGS. 8 and 9 is designed to be constant along longitudinal axis 62. This is moment is defined as the integral of the product of incremental areas dA of the cross section multiplied by the square of their distance R from the axis 66.

FIGS. 11A and 11B show a front elevational and a plan view, respectively, of a glabellar plate 12 manufactured according to the present invention. The plate is configured in the face plane 52 to be generally U-shaped. It is also configured in the contour dimension 56 and the cross-sectional dimension 54 to conform to the skeleton. The contour dimension 56 configuration and the cross-sectional 54 dimension twist are also shown in FIG. 11B, the plan view of glabellar plate 12. FIGS. 11A and 11B demonstrate the subtlety and complexity of the curves required in the glabellar plate 12 to conform to the glabella.

FIGS. 12A and 12B show the more radically configured medial canthal plate 20, while FIG. 13 shows the nasofrontal separation plate 16. The lateral buttress plate 26, which is also radically configured, is shown in FIGS. 14A and 14B. These figures demonstrate the advantages to be gained by preconfiguring bone stabilization plates 8; curves in the plate are more continuous to reduce facial distortion in the patient, time is saved in the operating room, and the plates are subjected to far less metal fatigue and crimping because they need to be bent and twisted less in the operating room than previous plates. Plates 8 can also be made of harder and stiffer material because they require less bending in the operating room.

FIGS. 15-20 show various face plane configurations for segments 58 and throat sections 60 of plates 8.

The parts of the present invention allow for efficient and effective treatment. The surgeon evaluates the injury to determine which sets 74 of plates 8 are required. If the evaluation is inaccurate or incomplete, additional presterilized packages can be obtained immediately without the need to wait while additional plates are sterilized. The fracture is exposed and reduced. The surgeon determines which plates are to be applied.

The surgeon then performs any minor bending or crimping that may be required finally to tailor the contour of the plate to the skeleton to which it will be affixed.

## Claims

1. A method of manufacturing an osteosynthesis plate comprising the steps of:
(a) selecting a craniofacial stabilization site on the human skull which is desired to be stabilized using an osteosynthesis plate;
(b) preparing a stamp that has been contoured in three dimensions to fit the structure and shape of craniofacial sites on a plurality of human skulls whose locations on the skulls correspond to the location of the craniofacial stabilization site;
(c) providing material for forming the plate;
(d) stamping the material to form an osteosynthesis plate that is contoured in three dimensions to fit the structure and shape of the craniofacial sites on the plurality of human skulls corresponding to the craniofacial stabilization site; and
(e) wherein the osteosynthesis plate is designed so that its cross sectional moment of inertia is constant and/or so that its polar moment of inertia is constant.

## Patentansprüche

1. Verfahren zur Herstellung einer Osteosynthese-Platte (einer Platte zum mechanischen Verbinden von Frakturenden), mit den Schritten:
(a) Auswählen einer das Gesicht oder den Schädel betreffenden (kraniofazialen) Stelle des menschlichen Schädels, die mittels einer Osteosynthese-Platte stabilisiert werden soll,
(b) Vorbereiten eines Stempels bzw. einer Form, der bzw. die dreidimensional geformt ist, um zu der Struktur and Form von das Gesicht oder den Schädel betreffenden (kraniofazialen) Stellen einer Vielzahl menschlicher Schädel zu passen, deren Lagen auf den Schädeln der Lage der zu stabilisierenden des Gesicht oder den Schädel betreffenden (kraniofazialen) Stelle entspricht,
(c) Vorsehen von Material zum Formen der Platte,
(d) Prägen des Materials zur Formung einer Osteosynthese-Platte, die dreidimensional geformt ist, im zu der Struktur und der Form der das Gesicht oder den Schädel betreffenden (kraniofazialen) Stellen der Vielzahl menschlicher Schädel zu passen, die der zu stabilisierenden das Gesicht oder den Schädel betreffenden (kraniofazialen) Stelle entsprechen und
(e) wobei die Osteosynthese-Platte so gestaltet ist, daß deren Trägheitsmoment des Querschnitts konstant ist und/oder deren polares Trägheitsmoment konstant ist.

## Revendications

1. Procédé de fabrication d'une plaque d'ostéosynthèse comprenant les étapes consistant à :
(a) sélectionner un site de stabilisation craniofacial sur le crâne humain que l'on souhaite stabiliser à l'aide d'une plaque d'ostéosynthèse ;
(b) préparer une matrice modelée en trois dimensions pour s'adapter à la structure et à la forme de sites craniofaciaux sur une pluralité de crânes humains dont les emplacements sur les crânes correspondent à l'emplacement du site de stabilisation craniofacial ;
(c) fournir un matériau pour former la plaque ;
(d) estamper le matériau pour former une plaque d'ostéosynthèse qui soit modelée en trois dimensions pour s'adapter à la structure et à la forme des sites craniofaciaux sur la pluralité de crânes humains correspondant au site de stabilisation craniofacial ; et
(e) dans lequel la plaque d'ostéosynthèse est conçue de sorte que son moment d'inertie en coupe transversale soit constant et/ou que son moment d'inertie polaire soit constant.
